(19) Europäisches Patentamt
European Patent Office
Office européen des brevets

(11) Veröffentlichungsnummer : **0 034 836**
**B1**

(12) **EUROPÄISCHE PATENTSCHRIFT**

(45) Veröffentlichungstag der Patentschrift : **13.07.83**

(51) Int. Cl.³ : **C 07 C107/06**, C 07 D249/20

(21) Anmeldenummer : **81102171.6**

(22) Anmeldetag : **18.06.79**

(60) Veröffentlichungsnummer der früheren Anmeldung nach Art. 76 EPÜ : **0006564**

(54) **Verfahren zum Herstellen von Nitroazobenzolen.**

(30) Priorität : **16.05.79 US 38768**

(43) Veröffentlichungstag der Anmeldung : **02.09.81 Patentblatt 81/35**

(45) Bekanntmachung des Hinweises auf die Patenterteilung : **13.07.83 Patentblatt 83/28**

(84) Benannte Vertragsstaaten : **BE DE FR GB IT**

(56) Entgegenhaltungen :
**DE A 2 334 769**

**Patents Abstracts of Japan Band 2, Nr. 131, 31. Oktober 1978 Seite 2822C78**

**HOUBEN-WEYL « Methoden der organischen Chemie » 4. Auflage, Band X/3, 1965, GEORG THIEME VERLAG, Stuttgart, Seiten 227 und 263**

(73) Patentinhaber : **CIBA-GEIGY AG**
**Patentabteilung Postfach**
**CH-4002 Basel (CH)**

(72) Erfinder : **Dexter, Martin**
***416 Cedar Drive West***
**Briarcliff Manor N.Y. 10510 (US)**
Erfinder : **Winter, Roland, A.E.**
**23 Banksville Road**
**Armonk New York 10504 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 034 836 B1

## Verfahren zum Herstellen von Nitroazobenzolen

Die Erfindung betrifft ein Verfahren zur Herstellung von o-Nitroazobenzol Zwischenprodukten, die in das entsprechende 2-Aryl-2H-benzotriazol umgewandelt werden können, welche ihrerseits als Lichtschutzmittel für organisches Material von grosser Bedeutung sind.

Das erfindungsgemässe Verfahren betrifft die Kupplung eines substituierten Phenols mit diazotiertem Anilin in einem stark alkalischen Alkanol. Ein Minimum an Wasser in der Reaktionsmischung ist vorteilhaft für die Löslichkeit des substituierten Phenols.

Das erfindungsgemässe Verfahren unterscheidet sich vom Stand der Technik in der Verwendung eines Ueberschusses an Alkalihydroxid, der weit über die stöchiometrische Menge hinausgeht, welche zum Neutralisieren des entstandenen Diazoniumsalzes und zur Salzbildung des Phenols notwendig ist. Demgemäss erfolgt die Kupplung in Gegenwart überschüssigen Hydroxyl- Ionen und bei einem pH-Wert von über 11.

Der Stand der Technik lehrt das Vermeiden von stark alkalischen Kupplungsprozessen. Daher ist der Erfolg, mit dem der gegenwärtige Kupplungsprozess durchgeführt wurde besonders überraschend.

Die Kupplung der Phenole mit Diazoniumsalz unter schwach alkalischen Bedingungen ist eine bekannte Methode für die Herstellung von aromatischen Azo-Derivaten. In « Basic Pripciples of Organic Chemistry » von J. D. Roberts und M. C. Caserio, W. A. Benjamin, Inc. New York (1965), S. 893-895 wird betont, dass der Kupplungsprozess bei etwa pH 10 optimal verläuft, bei höheren pH-Werten aber praktisch aufhört.

Ferner ist es bekannt, dass o-Nitrobenzoldiazoniumsalz-Lösungen in Gegenwart von Alkohol und Alkalimetallhydroxid instabil sind und unter $N_2$-Entwicklung sich schnell zersetzen. Aus diesem Grund lehrt der Stand der Technik Kupplungsreaktionen in überschüssigem Alkalihydroxid zu vermeiden. Siehe Fierz-David et al, « Fundamental Processes of Dye Chemistry », Interscience, New York (1949), S. 239-241, 252-253.

H. Zollinger « Azo and Diazo Chemistry », Interscience, New York (1961), S. 249-250 berichtet, dass sich bei stark alkalischer Kupplung das Gleichgewicht verschiebt von der Seite der Diazonium-Ionen.

SU-PS 360 357 beschreibt die Kupplung eines Natriumphenolates in schwach alkalischer Lösung mit überschüssigem sauren Diazoniumsalz, so dass der pH rapid unter den Wert 11 fällt während der Reaktion, ganz im Gegensatz zu den erfindungsmässigen Reaktionsbedingungen.

Im Gegensatz zu den Lehren des Standes der Technik o-Nitroazobenzol wird in hoher Ausbeute und in grosser Reinheit gewonnen, wenn die Reaktion in stark alkalischem Milieu und in Gegenwart eines niedrigen Alkanols als Lösungsmittel durchgeführt wird.

Die Erfindung betrifft daher ein Verfahren zur Herstellung von Verbindungen der Formel I

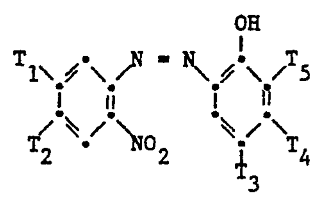

(I)

worin $T_1$ Wasserstoff oder Chlor ist, $T_2$ Wasserstoff, Chlor, $C_{1-4}$ Alkyl, $C_{1-4}$ Alkoxy, $C_{2-9}$ Carboalkoxy, Carboxy oder $—SO_3H$ ist, $T_3$ $C_{1-12}$ Alkyl, $C_{1-4}$ Alkoxy, Phenyl, Phenyl substituiert mit Alkyl-Gruppen, worin die Alkyl-Gruppen 1 bis 8 C-Atomen haben, Cycloalkyl mit 5 bis 6 C-Atomen, $C_{2-9}$ Carboalkoxy, Chlor, Carboxyäthyl oder $C_{7-9}$ Carboalkoxy, Chlor, Carboxyäthyl oder $C_{7-9}$ Arylalkyl ist. $T_4$ Wasserstoff, $C_{1-4}$ Alkyl, $C_{1-4}$ Alkoxy, Chlor oder Hydroxy ist und $T_5$ $C_{1-12}$ Alkyl, Chlor, $C_{5-6}$ Cycloalkyl oder $C_{7-9}$ Arylalkyl ist, dadurch gekennzeichnet, dass man das Diazoniumsalz eines Amins der Formel II

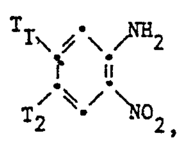

(II)

worin $T_1$ und $T_2$ die zuvor genannte Bedeutung haben, in stöchiometrischen Mengen oder in kleinem Ueberschuss, bezogen auf das zu kuppelnde Phenol, zu einer stark alkalischen niedrigen Alkanollösung oder deren wässrigen Lösung gibt, welche ein Phenol der Formel III

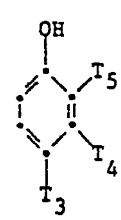

(III)

enthält, worin $T_3$, $T_4$ und $T_5$ die zuvor genannte Bedeutung haben, wobei die Phenollösung ein Alkalimetallhydroxid in solchen Mengen enthält, dass ein pH-Wert über 11 gesichert ist und auch nach Neutralisieren des sauren Diazoniumsalzes ein Hydroxylion-Ueberschuss vorhanden ist, und die Reaktionstemperatur $-15\,°C$ bis $+30\,°C$ beträgt. Vorzugsweise beträgt die Temperatur $-2\,°C$ bis $+5\,°C$.

Nach Beendigung der Reaktion, wird das Reaktionsgemisch vorteilhafterweise mit Essigsäure oder mit einer Mineralsäure angesäuert und das Produkt durch Filtrieren isoliert.

$T_2$ kann $C_{1-4}$ Alkyl bedeuten, wie z. B. Methyl, Aethyl oder n-Butyl. $T_8$ kann auch $C_{1-4}$ Alkoxy bedeuten, wie z. B. Methoxy, Aethoxy oder n-Butoxy. $T_2$ kann auch $C_{2-9}$ Carboalkoxy bedeuten, z. B. Carbomethoxy, Carboäthoxy oder Carbo-n-octoxy.

$T_3$ kann $C_{1-2}$ Alkyl sein, z. B. Methyl, Aethyl, sec-Butyl, tert-Butyl, Amyl, tert-Octyl oder n-Dodecyl. $T_3$ kann $C_{1-4}$ Alkoxy sein z. B. Methoxy, Aethoxy oder n-Butoxy. $T_3$ kann Phenyl substituiert mit $C_{1-8}$ Alkyl-Gruppen bedeuten, wobei die Alkyl-Gruppen z. B. Methyl, tert-Butyl, tert-Amyl oder tert-Octyl sein können. $T_3$ kann $C_{5-6}$ Cycloalkyl sein, z. B. Cyclopentyl oder Cyclohexyl. $T_3$ kann $C_{2-9}$ Carboalkoxy sein, z. B. Carbomethoxy, Carboäthoxy, Carbo-n-Butoxy oder Carbo-n-octoxy. $T_3$ kann $C_{7-9}$ Arylalkyl sein, z. B. Benzyl, α-Methylbenzyl oder α,α-Dimethylbenzyl.

$T_4$ kann $C_{1-4}$ Alkyl sein, z. B. Methyl, Aethyl oder n-Butyl.

$T_4$ kann $C_{1-4}$ Alkoxy sein, z. B. Methoxy, Aethoxy oder n-Butyloxy.

$T_5$ kann $C_{1-8}$ Alkyl sein, z. B. Methyl, sec-Butyl, tert-Butyl, tert-Amyl oder tert-Octyl.

$T_5$ kann $C_{5-6}$ Cycloalkyl sein, z. B. Cyclopentyl oder Cyclohexyl. $T_5$ kann $C_{7-9}$ Arylalkyl sein, z. B. Benzyl, α-Methylbenzyl oder α,α-Dimethylbenzyl.

Bevorzugt ist $T_1$ Wasserstoff.

Bevorzugt ist $T_2$ Wasserstoff, $C_{1-2}$ Alkyl, Methoxy oder Carboxy.

Bevorzugt ist $T_3$ $C_{1-12}$ Alkyl, Cycloalkyl-Phenyl, α-Methylbenzyl, α,α-Dimethylbenzyl oder Carboxyäthyl.

Bevorzugt ist $T_4$ Wasserstoff, Hydroxy oder Methyl.

Bevorzugt ist $T_5$ $C_{1-12}$ Alkyl, Cyclohexyl, Benzyl, α-Methylbenzyl oder α,α-Dimethylbenzyl.

Falls $T_3$ und $T_5$ beide Alkyl-Reste sind, so ist die Summe deren C-Atomen vorzugsweise 4.

Besonders bevorzugt ist $T_2$ Wasserstoff oder Chlor.

Besonders bevorzugt ist $T_3$ Methyl, tert-Butyl, tert-Amyl, tert-Octyl, sec-Butyl, Cyclohexyl, Carboxyäthyl, α-Methylbenzyl oder α,α-Dimethylbenzyl.

Besonders bevorzugt ist $T_4$ Wasserstoff.

Besonders bevorzugt ist $T_5$ Methyl, sec-Butyl, tert-Butyl, tert-Amyl, tert-Octyl, α-Methylbenzyl oder α,α-Dimethylbenzyl.

Die erfindungsgemässe alkalische Kupplung eignet sicht gut zur Herstellung des o-Nitroazobenzol Zwischenproduktes, welches durch die konventionelle sauere Kupplung nur in niedriger Ausbeute erhältlich ist. Das erfindungsgemässe Verfahren ist daher besonders geeignet für die Herstellung von o-Nitroazobenzolen, bei denen $T_3$ und $T_5$ Aralkyl-Reste sind, wie z. B. α-Methylbenzyl, α,α-Dimethylbenzyl oder tert.-Octyl.

Die Ausgangsprodukte, wie z. B. Phenole, o-Nitroanilin, 4-Chloro-2-nitroanilin oder α-Methylstyrol sind im Handel erhältlich oder nach bekannten Methoden auf einfache Weise herzustellen.

Die o-Nitrobenzoldiazonium-Verbindung wird wiederum durch übliche Diazotierungsverfahren hergestellt, wobei Natriumnitrit in saurer Lösung auf o-Nitroanilin der Formel II

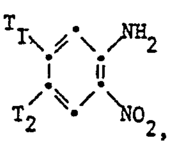

(II)

einwirkt.

Die Verbindungen der Formel II und III werden nachstehend mit einigen Beispielen illustriert. Sie alle sind im Handel erhältlich.

Phenole

2,4-Di-tert-Butylphenol
2,4-Di-tert-Amylphenol
2,4-Di-tert-Octylphenol
2-tert-Butyl-4-methylphenol
2,4-Di(α,α-dimethylbenzyl)phenol
2,4-Di-n-octylphenol
2,4-Dimethyl-phenol
3,4-Dimethyl-phenol
2-Methyl-4-(α,α-dimethylbenzyl)phenol
2,4-Dichlor-phenol

3,4-Dichlor-phenol
2-(α,α-Dimethyl-benzyl)-4-methylphenol
2-(α,α-Dimethyl-benzyl)-4-tert-butylphenol
2-(α,α-Dimethyl-benzyl-4-tert-octylphenol
2-tert-Octyl-4-(α,α-dimethyl-benzyl)phenol
2-(α-Methylbenzyl)-4-methylphenol
2-Cyclohexyl-4-methylphenol
2-sec-Butyl-4-tert-butylphenol
2-tert-Butyl-4-sec-butylphenol
2-Methyl-4-carboxyäthylphenol

Bevorzugte Phenole sind :

2,4-Di-tert-butylphenol
2,4-Di-tert-amylphenol
2,4-Di-tert-octylphenol
2-tert-Butyl-4-methylphenol
2-sec-Butyl-4-tert-butylphenol
2-(α-Methylbenzyl)-4-methylphenol
2,4-Di(α,α-dimethyl-benzyl)phenol
2-(α,α-Dimethyl-benzyl)-4-tert-octylphenol
2-tert-Octyl-4-(α,α-dimethyl-benzyl)phenol.

o-Nitroaniline

o-Nitroanilin
4-Chlor-2-nitroanilin
4,5-Dichlor-2-nitroanilin
4-Methoxy-2-nitroanilin
4-Methyl-2-nitroanilin
4-Aethyl-2-nitroanilin
n-Butyl-3-nitro-4-aminobenzoat
n-Octyl-3-nitro-4-aminobenzoat
4-n-Butoxy-2-nitroanilin
3-Nitro-4-aminobenzolsäure
3-Nitro-4-aminobenzolsulfonsäure

Bevorzugte o-Nitroaniline der Erfindung sind o-Nitroanilin und 4-Chlor-2-nitroanilin.

Die Umsetzung erfolgt indem man eine verdünnte sauere Lösung, vorzugsweise mit HCl angesäuert, welche das Diazoniumsalz des entsprechenden o-Nitroanilins enthält, zu einer stark alkalischen Lösung des entsprechenden Phenols gibt. Als niedrige Alkanole kommen Alkanole mit 1 bis 4 C-Atomen in Frage, wie z. B. Methanol, Aethanol, Isopropanol und n-Butanol, aber bevorzugt sind Methanol, Aethanol und Isopropanol, besonders bevorzugt ist Methanol.

Während der Kupplung sollte so wenig Wasser wie möglich vorhanden sein. Es ist von Vorteil, eine hochkonzentrierte Diazoniumsalz-Lösung zu verwenden, sowie eine alkalische Phenol-Lösung, welche nur ein niedriges Alkanol oder ein verdünntes Alkanol mit einem Minimum an Wassergehalt aufweist, einzusetzen. Das Lösungsmittelgemisch besteht aus mindestens 50 Gew.% Alkanol, vorzugsweise aus 75 Gew.%.

Die alkalische Alkanol-Lösung enthält ein Alkalimetall-Hydroxid, wie z. B. NaOH und KOH, vorzugsweise NaOH. Die Menge des Alkalimetall-Hydroxids wird so gewählt, dass nach Neutralisieren des saueren Diazoniumsalzes, die Hydroxylion-Konzentration im Reaktionsgemisch immer noch über pH 11 ist.

Grundsätzlich kann eine stöchiometrische Menge des Diazoniumsalzes, bezogen auf das Phenol, eingesetzt werden, ein kleiner Ueberschuss (bis zu 20 %) ist jedoch vorteilhaft. Es erleichtert einerseits das Isolieren des Produktes und erhöht anderseits die Ausbeute.

Die gemäss dem erfinderischen Verfahren erhaltenen Nitroazobenzole können auf an sich bekannte Weise durch reduktive Cyclisierung in die entsprechenden Benztriazole überführt werden. Als Reduktionssysteme eignen sich z. B. Zink und NaOH, Hydrazin oder katalytische Hydrierung mit einem Edelmetall-Katalysator. Die Verwendung dieser Mittel führt zu guten Benztriazolausbeuten.

Die folgenden Beispiele erläutern die Erfindung. Prozente bedeuten Gewichtsprozente.

A. Herstellung des Vorproduktes : 2,4-Di-(α,α-dimethyl-benzyl)phenol

Dieses Zwischenprodukt wurde nach der Beschreibung in US-PS 2 714 120 hergestellt. 705,8 g (7,5 Mol) Phenol wird mit 1 772,7 g (15 Mol) α-Methylstyrol in Gegenwart von 25,7 g (0,135 Mol) p-Toluolsulfonsäure Monohydrat als Katalysator vermischt und während 2,5 Std. auf 140 °C unter $N_2$ erhitzt. Nach Beendigung der Reaktions kühlt man das Gemisch auf 110 °C ab und fügt 1 125 ml Toluol

zu. Dann wäscht man die resultierende Lösung bei 80 °C mit 750 ml wässrige Lösung bestehend aus 37,5 g $NaCO_3$ und 75 g NaCl. Die organische Phase wäscht man dreimal mit 1 000 ml wässriger NaCl-Lösung, trocknet über wasserfreiem Natriumsulfat und anschliessend isoliert man durch Filtrieren und Vakuumdestillieren.

Die Hauptfraktion enthält 1 229,8 g des Produktes (49,6 % der Theorie). Sdp. 172-175°/0,15-0,18 mm/Hg. Smp. 63-65 °C.

B. Vergleichsbeispiel : Herstellung des Zwischenproduktes 2-Nitro-2′-hydroxy-3′,5′-di-(α,α-dimethyl-benzyl)azobenzol durch sauere Kupplung

In einen 2 l Dreihals-Rundkolben mit Rührer und Thermometer gibt man 90,6 g einer 26 %igen wässrigen Naphtalinsulfonsäure-Lösung, 1,9 g Triton X-207 (nicht ionischer Oberflächenaktiver Stoff), 5,6 g Conoco AAS-90F (Natrium-dodecylbenzol-sulfonat) und 90 ml Wasser. Das Gemisch wird auf 40 °C erwärmt, dann mit 116,5 g 2,4-Di-(α,α-dimethyl-benzyl)phenol, welches auf 90 °C vorgeheizt wurde, versetzt und unter kräftigem Rühren auf 40 °C gehalten.

Eine kalte o-Nitrobenzoldiazoniumchlorid-Lösung wird aus 49,8 g (0,36 Mol) o-Nitroanilin und aus 24,9 g (0,36 Mol) Natriumnitrit in conc. HCl bei – 5 °C bis 0 °C hergestellt. Diese Lösung wird tropfenweise während 3 Stunden zum Reaktionsgemisch gegeben und die resultierende dunkelrote bis schwarze Lösung über Nacht bei 40 °C gehalten. Dann wird die Temperatur für eine Stunde auf 65 °C und auf weitere 30 Minuten auf 95 °C erhöht. Nach Abkühlen auf 85 °C, wird das Rohprodukt, eine tiefrote viskose Masse, isoliert.

Das zerkleinerte Rohprodukt wird mit 200 ml heissem Wasser (75 °C) vermengt und anschliessend mit 400 ml Methanol über Nacht stehengelassen. Das Gemisch wird mit weiteren 400 ml Methanol zu einem feinen Granulat gerührt. Es ergab 81,9 g (48,4 % der Theorie) des Zwischenproduktes. Smp : 139-141 °C.

Der Dünnschichtchromatogramm zeigt ein homogenes Produkt mit $R_f = 0,61$ auf Silicagel in 3 Teilen Cyclohexan und 1 Teil Aethylacetat als Lösungsmittel.

## Beispiel 1

2-[2-Hydroxy-3,5-di-(α,α-dimethyl-benzyl)-phenyl]-2H-benzotriazol.

In einen 5 l Dreihals-Rundkolben mit Gaseinleitung, Rührer und Rückflusskühler gibt man 386 g (0,805 Mol) o-Nitroazobenzol, hergestellt im Beispiel A, und 1 200 ml Toluol. Zu dieser Lösung fügt man 240 ml Isopropanol und 240 ml Wasser. Unter Einleiten von Stickstoff fügt man 160 ml 50,1 % Natronlauge zur Lösung. Eine Flasche mit 158,2 g (2,42 Grammatom) Zink wird an den Reaktionskolben angeschlossen. Man leitet den Zinkstaub portionsweise während 90 Minuten in das Reaktionsgemisch, und zwar so, dass die Reaktionstemperatur zwischen 40-45 °C bleibt. Nach Beendigung der Zinkzugabe hält man das Reaktionsgemisch auf 40 °C 1 Stunde lang und erwärmt dann auf 70 °C 3 Stunden lang. Das Gemisch wird auf Raumtemperatur abgekühlt und mit 600 ml conc. Salzsäure angesäuert.

Der Zink-Rückstand wird durch Filtrieren entfernt. Die organische Phase, welche das Produkt enthält, wird viermal mit 340 ml verdünnter Salzsäure gewaschen. Nach Entfernen des Lösungsmittels durch Vakuumdestillation erhält man das viskose Rohprodukt, das beim Stehen kristallisiert.

Um das Rohprodukt zu reinigen rekristallisiert man es zuerst aus 750 ml Aethylacetat und dann aus 1 000 ml Acetonitril/Aethylacetat Gemisch (4 : 1) und löst anschliessend in 1250 ml Toluol. Die Toluol-Lösung wird mit 70 % wässriger Schwefelsäure extrahiert um farbige Verunreinigungen zu entfernen.

Man erhält 219,3 g (60,9 % der Theorie) gelblich-weisse Kristalle. Smp. 140-141 °C.

Analyse : $C_{30}H_{29}N_3O$

| | | | |
|---|---|---|---|
| berechnet | C : 80,51 | H : 6,53 | N : 9,39 |
| gefunden | C : 80,53 | H : 6,54 | N : 9,51 |

## Beispiel 2

2-Nitro-2′-hydroxy-3′,5′-di-(α,α-dimethyl-benzyl)-azobenzol

In einem 500 ml Dreihals-Kolben mit Rührer, Gaseinleitung und Pberdrucksicherung bigt man 13,5 g (0,21 Möl) festes Kaliumhydroxid und 10 ml Wasser. Die resultierende heisse Lösung wird mit 80 ml Methanol abgekühlt.

16,5 g (0,05 Mol) 2,4-Di-(α,α-dimethyl-benzyl)-phenol und 85 ml Methanol werden unter Stickstoff zugefügt und auf – 4 °C abgekühlt. Eine gekühlte Lösung enthaltend 42,9 g (0,06 Mol) o-Nitrobenzoldiazoniumchlorid in conc. HCl wird während 15 Minuten unter Rühren zugefügt und die Temperatur auf – 2 °C bis 0 °C gehalten. Sofort entsteht eine tiefrote Färbung, verursacht vom gebildeten Azofarbstoff-Phenoxid. Das Gemisch wird bei – 1 °C bis + 1 °C 10 Minuten lang weitergerührt, anschliessend mit 20 ml Eisessig langsam (während 2 Minuten) angesäuert und die Temperatur bei + 1 bis + 3 °C gehalten. Die

ziegelrote Suspension wird 15 Minuten lang weitergerührt, wobei eine Temperatursteigerung möglich ist, und anschliessend abfiltriert. Man wäscht den Filterrückstand mit einer Lösung aus 40 g Eis und 160 ml Methanol und zum Schluss mit 1 800 ml Wasser.

Das hellrote Rohprodukt wird im Vakuum bei 60 °C und 75 mm Hg während 16 Stunden getrocknet. Dies Ausbeute beträgt 22,7 g, 82 % der Theorie. Die spectrophotometrische Analyse zeigt 86,7 % Reinheit an. Smp : 135-140 °C.

Das Rohprodukt wird aus heissem n-Butanol (5 ml pro Gramm) umkristallisiert. Smp : 147-148 °C.

Beispiel 3

2-Nitro-2'-hydroxy-3',5'-di-(α,α-dimethyl-benzyl)-azobenzol

Unter Verwendung des Verfahrens von Beispiel 2, werden 13,6 g (0,34 Mol) feste Natrolauge in 145 ml Methanol gelöst, 16,5 g (0,05 Mol) 2,4-Di-(α,α-dimethyl-benzyl)-phenol und 20 ml Methanol zugefügt. Man kühlt die Lösung auf + 2 °C ab. Inzwischen wird eine o-Nitrobenzoldiazoniumchlorid-Lösung aus 8,3 g (0,06 Mol) o-Nitroanilin und 17,3 g conc. HCl sowie 6 ml Wasser und aus 4,3 g Natriumnitrit in 8 ml Wasser hergestellt. Man gibt die Diazonium-Lösung 2 Stunden lang bei + 2 °C zu der alkalischen Lösung. Das tiefrote Gemisch wird 30 Minuten lang bei + 2 °C gerührt, dann mit 20 ml Eisessig angesäuert und der hellrote Niederschlag durch Filtrieren isoliert. Man wäscht das Produkt mit 3 × 50 ml Methanol, dann mit 4 × 75 ml Wasser und anschliessend trocknet man bei 75 °C mm Hg. Die Ausbeute beträgt 21,6 g (89 %) mit 97 % Reinheit. Smp : 140-142 °C.

Beispiele 4-16

Alkalische Kupplung

Verfahren wird wie in Beispiel 2. Man verwendet jedoch anstelle von 2,4-Di-(α,α-dimethyl-benzyl)-phenol andere Phenole. Die Ausbeute variiert entsprechend den Substituenten am Phenol.

| Beispiel Nr. | Phenole | Ausbeute o-Nitroazobenzol in % |
|---|---|---|
| 4 | 2,4-Di-tert-amyl | 66 |
| 5 | 2,4-Di-tert-octyl | 70(50)[a] |
| 6 | 2,4-Di-tert-octyl | 47[b] |
| 7 | 2,4-Di-(α,α-Dimethyl-benzyl) | 82 |
| 8 | 2,4-Di-(α,α-Dimethyl-benzyl) | 89[c] |
| 9 | 2-(1-Phenyläthyl)-4-methyl | 84.3 |
| 10 | 4-Methyl | 56[d] |
| 11 | 2,4-Di-n-octyl | 56[e] |
| 12 | 2-Methyl-4-α,α-dimethyl-benzyl | 85* |
| 13 | 2-α,α-Dimethyl-benzyl-4-methyl | 66[f] |
| 14 | 2-(α,α-Dimethyl-benzyl)-4-tert-butyl | 54 |
| 15 | 2-(α,α-Dimethyl-benzyl)-4-tert-octyl | 62 |
| 16 | 2-tert-Octyl-4-(α,α-dimethyl-benzyl) | 73 |
| | Kontrolle ohne Phenol | 0[g] |

a) Verfahren wurde wie im Beispiel 3 mit hoher Alkalikonzentration. Die Ausbeute beträgt nach der Reinigung 50 %. $N_2$ Gasentwicklung fand statt. (= 48 % der Theorie).
b) $N_2$ Entwicklung = 60 % der Theorie.
c) Verfahren wurde nach Beispiel 3.
d) $N_2$ Gasentwicklung = 29 % der Theorie.
e) Die sauere Kupplung ergab nur 16-19 % Ausbeute.
f) $N_2$ Gasentwicklung = 33 % der Theorie.
g) Wenn kein Phenol vorhanden, etwa 67 % der theoretisch vorhandenen $N_2$ entwickelt sich aus der zersetzten Diazoniumlösung.
* Die Ausbeute beträgt 47 % wenn 4-Chloro-2-nitrobenzoldiazoniumchlorid mit diesem Phenol gekuppelt wird.

Beispiel 17

2-Nitro-2'-hydroxy-3',5'-di-(α,α-dimethyl-benzyl)-azobenzol

Wenn man im Beispiel 2 anstelle von Methanol Aethanol verwendet, wird eine $N_2$-Entwicklung (50 %

der Theorie) beobachtet. Man erhält eine niedrige Ausbeute (50,5 %) des oben genannten Produktes. Zum gleichen Ergebnis führt, wenn man anstelle von Methanol Isopropanol verwendet.

Diese Daten zeigen, dass Methanol das geeigneteste Lösungsmittel für die alkalische Kupplung ist.

Beispiel 18

4-Chloro-2-nitro-2'-hydroxy-3',5'-di-(α,α-dimethylbenzyl)-azobenzol

Wenn man im Vergleichsbeispiel B anstelle von der Diazoniumlösung, hergestellt aus 2-Nitroanilin, eine Diazoniumlösung, hergestellt aus 4-Chloro-2-nitroanilin in äquivalenter Menge verwendet, erhält man 47,3 % vom dunkelroten Produkt.

Beispiel 19

2-Nitro-2'-hydroxy-3'-α,α-dimethylbenzyl-5-tert-octylazobenzol.

Diese Verbindung wird nach dem allgemeinen Verfahren des Beispiels 3 erhalten durch Kupplung von o-Nitrobenzol-diazonium-Chlorid mit 2-α, -Dimethylbenzyl-4-tert-octyl-phenol, als roter kristalliner Feststoff, F 133-134 °C.

**Ansprüche**

1. Verfahren zur Herstellung von Verbindungen der Formel I

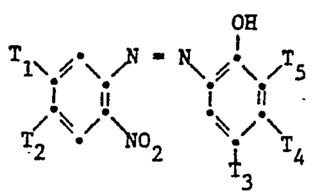

(I)

worin $T_1$ Wasserstoff oder Chlor ist, $T_2$ Wasserstoff, Chlor, $C_{1-4}$ Alkyl, $C_{1-4}$ Alkoxy, $C_{2-9}$ Carboalkoxy, Carboxy oder $—SO_3H$ ist, $T_3$ $C_{1-12}$ Alkyl, $C_{1-4}$ Alkoxy, Phenyl, Phenyl substituiert mit Alkyl-Gruppen, worin die Alkyl-Gruppen 1 bis 8 C-Atomen haben, Cycloalkyl mit 5 bis 6 C-Atomen, $C_{2-9}$ Carboalkoxy, Chlor, Carboxyäthyl oder $C_{7-9}$ Carboalkoxy, Chlor, Carboxyäthyl oder $C_{7-9}$ Arylalkyl ist, $T_4$ Wasserstoff, $C_{1-4}$ Alkyl, $C_{1-4}$ Alkoxy, Chlor oder Hydroxy ist und $T_5$ $C_{1-12}$ Alkyl, Chlor, $C_{5-6}$ Cycloalkyl oder $C_{7-9}$ Arylalkyl ist, dadurch gekennzeichnet, dass man das Diazoniumsalz eines Amins der Formel II

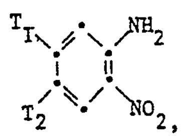

(II)

worin $T_1$ und $T_2$ die zuvor genannte Bedeutung haben, in stöchiometrischen Mengen oder in kleinem Ueberschuss, bezogen auf das zu kuppelnde Phenol, zu einer stark alkalischen niedrigen Alkanollösung oder deren wässrigen Lösung gibt, welche ein Phenol der Formel III

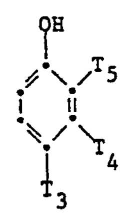

(III)

enthält, worin $T_3$, $T_4$ und $T_5$ die zuvor genannte Bedeutung haben, wobei die Phenollösung ein Alkalimetallhydroxid in solchen Mengen enthält, dass ein pH-Wert über 11 gesichert ist und auch nach Neutralisieren des sauren Diazoniumsalzes ein Hydroxylion-Ueberschuss vorhanden ist, und die Reaktionstemperatur – 15 °C bis + 30 °C beträgt und nach Ansäuern der Reaktionsmischung das Produkt isoliert.

2. Verfahren gemäss Anspruch 1 zur Herstellung einer Verbindung, worin $T_1$ Wasserstoff, $T_2$ Wasserstoff, $C_{1-2}$ Alkyl, Methoxy oder Carboxy, $T_3$ $C_{1-12}$ Alkyl, Cyclohexyl, Phenyl, α-Methylbenzyl, α,α-Dimethyl-benzyl oder Carboxyäthyl, $T_4$ Wasserstoff, Hydroxy oder Methyl, und $T_5$ $C_{1-12}$ Alkyl, Cyclohexyl, Benzyl, α-Methylbenzyl oder α,α-Dimethyl-benzyl ist.

3. Verfahren gemäss Anspruch 1 zur Herstellung der Verbindung 2-Nitro-2'-hydroxy-3',5'-di(α,α-dimethyl-benzyl)-azobenzol.

4. Verfahren gemäss Anspruch 1 zur Herstellung der Verbindung 2-Nitro-2'-hydroxy-3',5'-di-tert-octylazobenzol.

5. Verfahren gemäss Anspruch 1, dadurch gekennzeichnet, dass man nach beendigter Reaktion, die Verbindungen der Formel I durch reduktive Cyclisierung in bekannter Weise in die entsprechenden Benztriazole überführt.

## Claims

1. A process for the preparation of a compound of the formula VII

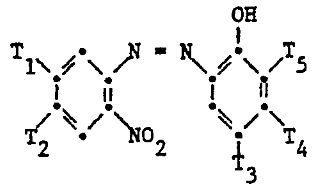

(I)

in which $T_1$ is hydrogen or chlorine, $T_2$ is hydrogen, chlorine, $C_{1-4}$ alkyl, $C_{1-4}$ alkoxy, $C_{2-9}$ carboalkoxy, carboxyl or $-SO_3H$, $T_3$ is $C_{1-12}$ alkyl, $C_{1-4}$ alkoxy, phenyl, phenyl substituted by alkyl groups, in which the alkyl groups have 1 to 8 C atoms, cycloalkyl having 5 to 6 C atoms, $C_{2-9}$ carboalkoxy, chlorine, carboxyethyl or $C_{7-9}$ arylalkyl, $T_4$ is hydrogen, $C_{1-4}$ alkyl, $C_{1-4}$ alkoxy, chlorine or hydroxyl and $T_5$ is $C_{1-12}$ alkyl, chlorine, $C_{5-6}$ cycloalkyl or $C_{7-9}$ arylalkyl, which comprises adding the diazonium salt of an amine of the formula VIII

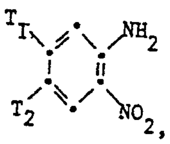

(II)

in which $T_1$ and $T_2$ are as defined above, in stoichiometric amounts or in a small excess, based on the phenol to be coupled, to a strongly alkaline lower alkanol solution, or an aqueous solution thereof, which contains a phenol of the formula IX

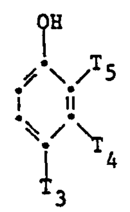

(III)

in which $T_3$, $T_4$ and $T_5$ are as defined above, the phenol solution containing an alkali metal hydroxide in amounts such that a pH value of above 11 is ensured and that excess hydroxyl ions are present even after neutralising the acid diazonium salt, and the reaction temperature being − 15 °C to + 30 °C, and isolating the product after acidifying the reaction mixture.

2. A process according to claim 1, for the preparation of a compound in which $T_1$ is hydrogen, $T_2$ is hydrogen, $C_{1-2}$ alkyl, methoxy or carboxyl, $T_3$ is $C_{1-12}$ alkyl, cyclohexyl, phenyl, α-methylbenzyl, α,α-dimethyl-benzyl or carboxyethyl, $T_4$ is hydrogen, hydroxyl or methyl and $T_5$ is $C_{1-12}$ alkyl, cyclohexyl, benzyl, α-methylbenzyl or α,α-dimethyl-benzyl.

3. A process according to claim 1, for the preparation of the compound 2-nitro-2'-hydroxy-3',5'-di-(α,α-dimethyl-benzyl)-azobenzene.

4. A process according to claim 1, for the preparation of the compound 2-nitro-2'-hydroxy-3',5'-di-tert-octylazobenzene.

5. A process according to claim 1, wherein the compound of the formula I is converted by reductive cyclisation in known manner, and when the reaction is complete, into the corresponding benztriazole.

## Revendications

1. Procédé de préparation de composés répondant à la formule (I)

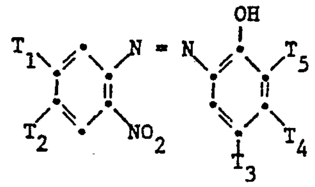

(I)

dans laquelle $T_1$ représente l'hydrogène ou le chlore, $T_2$ représente l'hydrogène, le chlore, un alkyle en $C_1$-$C_4$, un alcoxy en $C_1$-$C_4$, un alcoxycarbonyle en $C_2$-$C_9$, un carboxy ou un sulfo ($-SO_3H$), $T_3$ représente un alkyle en $C_1$-$C_{12}$, un alcoxy en $C_1$-$C_4$, un phényle, un phényle porteur de radicaux alkyles en $C_1$-$C_8$, un cycloalkyle en $C_5$ ou $C_6$, un alcoxycarbonyle en $C_2$-$C_9$, le chlore, un carboxyéthyle ou un arylalkyle en $C_7$-$C_9$, $T_4$ représente l'hydrogène, un alkyle en $C_1$-$C_4$, un alcoxy en $C_1$-$C_4$, le chlore ou un hydroxy, et $T_5$ représente un alkyle en $C_1$-$C_{12}$, le chlore, un cycloalkyle en $C_5$ ou $C_6$ ou un arylalkyle en $C_7$-$C_9$, procédé caractérisé en ce qu'on introduit le sel de diazonium d'une amine répondant à la formule (II)

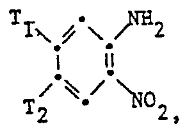

(II)

dans laquelle $T_1$ et $T_2$ ont les significations précédemment données, en une quantité stœchiométrique ou en un léger excès par rapport au phénol à copuler, dans une solution fortement alcaline, au sein d'un alcanol inférieur ou d'eau, qui contient un phénol répondant à la formule (III)

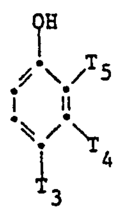

(III)

dans laquelle $T_3$, $T_4$ et $T_5$ ont les significations qui ont été données ci-dessus, la solution du phénol renfermant un hydroxyde de métal alcalin en une quantité telle qu'un pH supérieur à 11 soit garanti et que, même après neutralisation du sel de diazonium acide, il y ait un excès d'ions hydroxyles, et la température réactionnelle est comprise entre − 15 et + 30 °C, et, après acidification du mélange réactionnel, on isole le produit.

2. Procédé selon la revendication 1 pour la préparation d'un composé dans lequel $T_1$ représente l'hydrogène, $T_2$ représente l'hydrogène, un alkyle en $C_1$ ou $C_2$, un méthoxy ou un carboxy, $T_3$ représente un alkyle en $C_1$-$C_{12}$, un cyclohexyle, un phényle, un α-méthylbenzyle, un α, α-diméthylbenzyle ou un carboxyéthyle, $T_4$ représente l'hydrogène, un hydroxy ou un méthyle, et $T_5$ représente un alkyle en $C_1$-$C_{12}$, un cyclohexyle, un benzyle, un α-méthylbenzyle ou un α, α-diméthylbenzyle.

3. Procédé selon la revendication 1 pour la préparation du nitro-2 hydroxy-2′ bis-(α, α-diméthylbenzyl)-3′,5′ azobenzène.

4. Procédé selon la revendication 1 pour la préparation du nitro-2 hydroxy-2′ di-tert-octyl-3′, 5′ azobenzène.

5. Procédé selon la revendication 1, caractérisé en ce que, après la fin de la réaction, on transforme les composés de formule (I) par cyclisation réductrice, de manière connue, en les benzotriazoles correspondants.